# EUROPÄISCHE PATENTSCHRIFT

(11) **EP 4 514 433 B1**
(45) Veröffentlichungstag und Bekanntmachung des Hinweises auf die Patenterteilung: **25.02.2026**
(21) Anmeldenummer: 23718249.8
(22) Anmeldetag: 06.04.2023
(51) Int. Cl.: A61M 11/00, A61M 15/00, A61M 15/08, B05B 11/10, G16H 20/13

(54) **SPENDER**
DISPENSER
DISTRIBUTEUR

(30) Priorität: 26.04.2022 EP 22170084
(43) Veröffentlichungstag der Anmeldung: 05.03.2025
(73) Patentinhaber: Aptar Radolfzell GmbH, 78315 Radolfzell (DE)
(72) Erfinder: KOHNLE, Jörg, 78056 Villingen-Schwenningen (DE); HELMLINGER, Michael, 78315 Radolfzell-Böhringen (DE)
(74) Vertreter: Witte, Weller und Partner Patentanwälte mbB Stuttgart
(86) Internationale Anmeldenummer: PCT/EP2023/059212
(87) Internationale Veröffentlichungsnummer: WO 2023/208569

(56) Entgegenhaltungen:
- EP-A1- 4 169 500
- WO-A1-2014/004437
- WO-A1-2017/141194
- WO-A1-2021/175841
- WO-A1-2021/209320
- WO-A1-93/25841
- WO-A2-2015/193833
- DE-A1- 102019 217 937
- KR-B1- 101 994 297
- US-A- 3 584 348
- US-A1- 2013 076 514
- US-A1- 2015 024 335
- US-A1- 2017 246 406
- US-A1- 2019 307 648
- US-A1- 2020 288 788
- US-A1- 2021 090 703
- US-B2- 11 260 182

## Beschreibung

### ANWENDUNGSGEBIET UND STAND DER TECHNIK

Die Erfindung betrifft einen Spender zum Austrag pharmazeutischer oder kosmetischer Medien.

Ein gattungsgemäßer Spender dient dem Austrag von Medien, insbesondere von pharmazeutischen Flüssigkeiten. Es ist bekannt, solche Spender mit Auswerteeinheiten zu versehen, die über eine Sensorik die Nutzung des Spenders erfassen, beispielsweise um eine nachfolgende Prüfung zu gestatten, ob ein Medikament in richtiger Weise und in Übereinstimmung mit einem Medikationsplan eingenommen wird.

Bekannte Auswerteeinheiten sind in den jeweiligen Spender baulich integriert oder sind lösbar am Spender angebracht, so dass sie vom Spender gelöst und an einen anderen Spender gleichen Typs angebracht werden können.

Bei lösbaren Auswerteeinheiten ist ein Kopplungsmechanismus erforderlich, um sie am Gehäuse des Spenders anzubringen.

Die Dokumente WO 2014/004437 A1, US 2020/288788 A1, US 11 260 182 B2, US 2015/024335 A1 und KR 101 994 297 B1 offenbaren Spender mit magnetisch gekoppelten Auswerteeinheiten.

### AUFGABE UND LÖSUNG

Aufgabe der Erfindung ist es, eine Auswerteeinheit für einen Spender zur Verfügung zu stellen, die eine bequeme Handhabung im Zuge der Demontage und Montage an einem Spender gestattet.

Erfindungsgemäß wird ein Spender zum Austrag pharmazeutischer oder kosmetischer Medien vorgeschlagen, der über eine elektronische Auswerteeinheit verfügt. Die Erfindung betrifft darüber hinaus auch die Auswerteeinheit als solche.

Ein erfindungsgemäßer Spender weist ein Gehäuse auf, das die Außenkontur des Spenders bildet und mehrteilig ausgebildet sein kann. Insbesondere kann ein solches Gehäuse einen Speicherkörper aufweisen, der den Medienspeicher und insbesondere einen Flüssigkeitsspeicher bildet, in dem das Medium, insbesondere eine pharmazeutische Flüssigkeit, vor dem Austrag gelagert ist.

Der Spender weist eine Austragöffnung auf, durch die Medium aus dem Medienspeicher in eine umgebende Atmosphäre abgegeben werden kann.

Bevorzugt ist der Spender zur Ausbringung von Flüssigkeit ausgebildet, insbesondere in Form eines Strahls, eines Sprühstrahls, oder in Form von Einzeltropfen. Stattdessen kann der Spender jedoch auch für andere Medien vorgesehen sein, so insbesondere zur Ausbringung von Pulver zum Zwecke der Inhalation.

Ist der Spender als Flüssigkeitsspender ausgebildet, so kann es sich insbesondere um einen Pumpspender handeln, der über eine manuell betätigbare Pumpeinrichtung verfügt, die Flüssigkeit aus dem Flüssigkeitsspeicher zur Austragöffnung fördert. Alternativ handelt es sich um einen Quetschflaschenspender, der einen elastisch verformbaren Speicherkörper aufweist, durch dessen manuelle Komprimierung Flüssigkeit ausgetragen werden kann.

Der Spender kann weiterhin als Inhalator ausgebildet sein, insbesondere als Spender mit einem zur Abgabe einer Dosis der Flüssigkeit niederdrückbarem Container zur Abgabe einer definierten Dosis des Medikaments. Gemäß einem weiteren Beispiel kann der Spender als Nasalspender ausgebildet sein, insbesondere mit einem langgestreckten Nasalapplikator, an dessen distalem Ende die Austragöffnung vorgesehen ist.

Die elektronische Auswerteeinheit ist zur Erfassung der Nutzung des Spenders ausgebildet. Sie weist hierfür elektrische bzw. elektronische Komponenten auf, insbesondere eine Batterie, einen Prozessor und mindestens einen Sensor. Über den Sensor werden Daten erfasst, die Rückschlüsse auf die Spendernutzung zulassen. Die Sensoren können dabei dafür ausgebildet sein, unmittelbar eine externe Kraftbeaufschlagung zu erfassen, wie sie beispielsweise beim Niederdrücken einer Betätigungshandhabe zur Durchführung einer Pumpbetätigung auftritt. Vorzugsweise ist jedoch mindestens ein Sensor vorgesehen, der berührungslos und ohne externe Kraftbeaufschlagung Daten erfassen kann, wobei es sich insbesondere um ein Mikrofon und/oder einen Bewegungssensor handeln kann. Beispielsweise können die Ausgangsdaten eines Mikrofons ausgewertet werden, um charakteristische Geräusche während der Nutzung zu erkennen.

Die Auswerteeinheit umfasst vorzugsweise eine Ausgabeeinrichtung, insbesondere in Form mindestens einer LED, eines Displays, eines Vibrators und/oder eines Lautsprechers, um Signale an den Nutzer abzugeben, beispielsweise um die Information zu übermitteln, dass ein Austrag sensiert worden ist oder gemäß Medikationsplan ansteht.

Die Auswerteeinheit ist vorzugsweise dafür ausgebildet, zusammen mit einem externen Gerät wie insbesondere einem Smartphone verwendet zu werden. Zur Kommunikation mit dem externen Gerät weist die Auswerteeinheit vorzugsweise ein Funkmodul, insbesondere ein Bluetooth- und/oder WIFI-Funkmodul oder aber ein GSM-/3G-/4G- oder 5G-Funkmodel auf.

Um die Auswerteeinheit schnell und bequem vom Gehäuse des Spenders trennen und an einen neuen Spender ankoppeln zu können, ist erfindungsgemäß an der Außenseite des Gehäuses des Spenders, insbesondere an einem Speicherkörper des Gehäuses, eine magnetische oder magnetisierbare Haltefläche vorgesehen. Korrespondierend hierzu ist eine Anlageseite der Auswerteeinheit magnetisch oder magnetisierbar ausgebildet, so dass die Auswerteeinheit magnetisch am Gehäuse befestigt werden kann.

Die Anbringung der Auswerteeinheit ist durch die erfindungsgemäß vorgesehene Magnetverbindung sehr einfach und bequem möglich. Sobald die Auswerteeinheit in der Bereich des Gehäuses gebracht wird, der für die Anbringung der Auswerteeinheit vorgesehen ist, wird die Auswerteeinheit magnetisch angezogen und bleibt in der bestimmungsgemäßen Position am Gehäuse des Spenders haften.

Neben der Bequemlichkeit der Handhabung wird hierdurch bei geeigneter Gestaltung und insbesondere Größe der Haltefläche am Gehäuse auch erreicht, dass die Auswerteeinheit recht genau eine vordefinierte Position einnimmt. Dies stellt einen Vorteil dar, denn diese vordefinierte Position kann so gewählt sein, dass sie zur Erkennung der Nutzung des Spenders, insbesondere über einen Bewegungssensor oder ein Mikrofon, besonders gut geeignet ist, dass also die über den mindestens einen Sensor erfassbaren Daten, bspw. Geräusche während der Nutzung, einen sicheren Rückschluss auf die Nutzung des Spenders zulassen.

Vorzugsweise ist am Gehäuse eine magnetisierbare und insbesondere eine metallische Haltefläche vorgesehen, während an der Auswerteeinheit mindestens ein Permanentmagnet vorgesehen ist, aber auch die umgekehrte Konstellation ist möglich. Auch ist eine Gestaltung möglich, bei der sowohl die Haltefläche als auch die magnetische Gestaltung der Auswerteeinheit mit Permanentmagneten realisiert sind.

Als Permanentmagneten kommen insbesondere Neodym-Magneten in Frage, da solche Magneten eine starke Magnetkraft bereitstellen und daher für die sichere Befestigung der Auswerteeinheit am Gehäuse des Spenders besonders von Vorteil sind.

Die Auswerteeinheit ist vorzugsweise zur lösbaren Anbringung an einer Mantelfläche des Speicherkörper des Gehäuses vorgesehen. Der Speicherkörper selbst weist vorzugsweise im Bereich der Mantelfläche eine zylindrische Form auf, insbesondere mit einem kreisförmigen oder einem elliptischen Querschnitt.

Die Außenseite des Speicherkörpers kann mit der magnetischen oder magnetisierbaren Haltefläche versehen sein. Entsprechend der gewölbten und kreisabschnittsförmigen oder zylinderabschnittsförmigen Mantelfläche ist vorzugsweise auch die magnetische oder magnetisierbare Haltefläche gewölbt ausgebildet.

Die Auswerteinheit weist vorzugsweise ein Elektronikgehäuse auf, welches die Anlageseite bildet und innerhalb dessen die elektronischen Komponenten der Auswerteinheit angeordnet sind, insbesondere also ein Prozessor, ein Speicher und eine Batterie sowie je nach Typ der Auswertung einen oder mehrere Sensoren. Dieses Elektronikgehäuse umgibt das Gehäuse des Spenders und insbesondere den Speicherkörper nicht vollständig, sondern ist vorzugsweise rucksackähnlich an einer Seite des Speicherkörpers vorgesehen, so dass der Spender einschließlich der Auswerteeinheit hierdurch eine asymmetrische Formgebung erhält. Insbesondere vorzugsweise überdeckt das Elektronikgehäuse in Umfangsrichtung höchstens 50% der Mantelfläche des Speicherkörpers, vorzugsweise höchstens 40%.

Der Speicherkörper weist vorzugsweise Kunststoff als Hauptmaterial auf. Die magnetische oder magnetisierbare Haltefläche ist vorzugsweise als separates Teil ausgebildet, insbesondere als zumindest partiell metallisches Teil, welches außenseitig an einer Kunststoffwandung des Speicherkörpers fest angebracht ist. Entsprechend der oben genannten Erstreckung des Elektronikgehäuses umgibt auch diese Haltefläche die Mantelfläche vorzugsweise nur partiell, insbesondere zu maximal 50%, insbesondere vorzugsweise zu maximal 40%.

Die am Gehäuse vorgesehene Haltefläche kann auf verschiedene Arten bereitgestellt werden. Erfindungsgemäß ist die Haltefläche im Bereich eines beschrifteten Etiketts vorgesehen. Sie kann unmittelbar das Etikett bilden oder mittels des Etiketts an einer Gehäusefläche des Gehäuses festgeklebt sein. Das Etikett kann die Produktbezeichnung oder eine Produktbeschreibung zu dem im Medienspeicher enthaltenen pharmazeutischen oder kosmetischen Produkt umfassen. Alternativ kann das Etikett auch eine Handhabungsanleitung enthalten, die die Anbringung der Auswerteeinheit beschreibt. Diese kann auch beispielsweise in einem Symbol bestehen, welches die Anbringung verdeutlicht.

Die Haltefläche kann, in nicht beanspruchten Ausführungsformen, auch unabhängig vom Etikett selbstständig am Gehäuse mittels einer Klebeverbindung angebracht sein. Auch sind nicht beanspruchte Gestaltungen möglich, bei denen die Haltefläche durch eine Haltehülse oder Halteklemme gebildet wird, die klemmend am Gehäuse des Spenders angebracht ist. Eine solche Haltehülse oder Halteklemme kann insbesondere vollständig oder nahezu vollständig metallisch sein. Sie kann jedoch auch aus Kunststoff gefertigt sein und einen magnetischen oder magnetisierbaren Einsatz aufweisen.

Gemäß einem zweiten, nicht beanspruchten Aspekt der Offenbarung wird eine alternative Gestaltung vorgeschlagen, die die bequeme Anbringung der Auswerteeinheit am Gehäuse des Spenders gestattet. Gemäß dieser alternativen Gestaltung ist vorgesehen, dass die Auswerteeinheit mindestens zwei und vorzugsweise mindestens drei elastisch auslenkbare Haltearme aufweist, die das Gehäuse umgreifen und damit einen klemmenden Halt der Auswerteinheit am Gehäuse bewirken.

Die Haltearme erstrecken sich vorzugsweise in unterschiedlichen Richtungen von einem Gehäuse der Auswerteeinheit um das Spendergehäuse herum. Die Haltearme definieren im entspannten Zustand einen lichten Querschnitt, der kleiner als der Querschnitt des Gehäuses und insbesondere des Speicherkörpers ist, so dass sie nach Anbringung am Gehäuse unter elastischer Spannung stehen. Zur Einbringung des Gehäuses in den lichten Querschnitt werden die Haltearme vorzugsweise aufgebogen. Dies kann unmittelbar manuell an den Haltearmen erfolgen oder durch mit den Haltearmen verbundene Greifflächen geschehen, die bei Kraftbeaufschlagu2ng mittelbar die Haltearme öffnen und den lichten Querschnitt dadurch vergrößern.

Die Haltearme lassen sich flexibel auslenken, so dass sie eine Kopplung an Speicherkörper unterschiedlicher Querschnittsform und insbesondere unterschiedlicher Durchmesser gestatten.

Die elektronische Auswerteeinheit ist vorzugsweise zur lösbaren Anbringung an einer Mantelfläche des Speicherkörper des Gehäuses vorgesehen. Der Speicherkörper selbst weist vorzugsweise im Bereich der Mantelfläche eine zylindrische Form auf, insbesondere mit einem kreisförmigen oder einem elliptischen Querschnitt.

Die Auswerteinheit weist vorzugsweise ein Elektronikgehäuse auf, innerhalb dessen die elektronischen Komponenten der Auswerteinheit vorgesehen sind. Von diesem Elektronikgehäuse aus erstrecken sich die Haltearme vorzugsweise in Umfangsrichtung um die Mantelfläche des Speicherköpers herum, wobei die Haltearme jeweils einen Winkelbereich von mindestens 120° überspannen. Die Haltearme sind mit ihrer jeweiligen Wurzel an dem genannten Elektronikgehäuse fest angebracht und erzielen ihre Flexibilität darüber, dass sie in sich elastisch verformbar sind.

Die einzelnen Haltearme sind vorzugsweise gekrümmt. Insbesondere erstrecken sie sich kreisabschnittsförmig, um einen in etwa kreiszylindrischen Gehäuseabschnitt zu umgreifen, insbesondere einen entsprechend geformten Speicherkörper. Die Haltearme erstrecken sich vorzugsweise über einen Winkelbereich von mindestens 120°, so dass zwei in entgegengesetzte Richtung das Gehäuse umgebende Haltearme das Gehäuse um mindestens etwa 240° umgreifen. Die Haltearme umgeben das Gehäuse und insbesondere den Speicherkörper nicht vollständig. Vorzugsweise umgeben die Haltearme das Gehäuse bzw. den Speicherkörper zu maximal 75%, weisen also eine Umfangserstreckung von maximal 270° auf.

Der Durchmesser des Grundkreises der Kreisabschnittsform, entsprechend derer die Haltearme geformt sind, beträgt vorzugsweise zwischen 10 mm und 25 mm.

Die Zahl der Haltearme beträgt vorzugsweise mindestens drei, wobei von diesen drei Haltearmen vorzugsweise zwei sich in einer ersten Erstreckungsrichtung um das Spendergehäuse herum erstrecken, während der dritte Haltearm zwischen diesen beiden Haltearmen angeordnet ist und sich in entgegengesetzter Erstreckungsrichtung um das Spendergehäuse herum erstreckt.

Unabhängig von der Anbringungsmethode über Magnetismus oder über die Haltearme kann die Auswerteeinheit ein Gehäuse aufweisen, welches nicht-modular aufgebaut ist und für den Anwender nicht weiter bestimmungsgemäß zerlegbar ist. Eine besondere erfindungsgemäße Ausgestaltung eines Spenders und einer Auswerteeinheit sieht jedoch vor, dass die Auswerteeinheit eine Befestigungseinheit und eine Elektronikeinheit umfasst, die getrennte Baueinheiten darstellen.

Die Befestigungseinheit ist dabei zur lösbaren Anbringung am Gehäuse ausgebildet und weist hierfür die beschriebene magnetische oder magnetisierbare Gestaltung und/oder die beschriebenen Haltearme auf. Die hiervon trennbare Elektronikeinheit ist zur lösbaren Anbringung an der Befestigungseinheit vorgesehen. Die Elektronikeinheit umfasst vorzugsweise die Gesamtheit der elektronischen Komponenten der Auswerteeinheit, mindestens jedoch die Batterie, den Prozessor sowie mindestens einen Sensor.

Die Zweiteilung der Auswerteeinheit gestattet es, die Elektronikeinheit mit unterschiedlichen Befestigungseinheiten zu verwenden, beispielsweise solchen zur magnetischen Befestigung am Spendergehäuse und solchen zur Befestigung am Spendergehäuse mit oben beschriebenen Haltearmen.

Die gleiche Elektronikeinheit kann demnach durch Konfiguration mit unterschiedlichen Befestigungseinheiten zur Anbringung an vielen unterschiedlichen Spendern vorgesehen sein.

Die Möglichkeit, die Befestigungseinheit und die Elektronikeinheit zu trennen und in anderer Konfiguration wieder zusammenzusetzen, muss nicht zwingend für den Endanwender gegeben sein. Die genannte Modularität kann auch dafür genutzt werden, um einheitliche Elektronikeinheiten bereits im Rahmen der Herstellung und Bereitstellung bedarfsgerecht mit passenden Befestigungseinheiten zu versehen und ggf. fest zu verbinden.

Besonders bevorzugt ist es allerdings, wenn die Befestigungseinheit zur werkzeuglos lösbaren Anbringung an der Elektronikeinheit ausgebildet ist und damit dem Endanwender selbst die Möglichkeit des Wechsels der Befestigungseinheit offensteht. Insbesondere kann zu diesem Zweck vorgesehen sein, dass die Elektronikeinheit magnetisch an der Befestigungseinheit befestigt ist oder die Elektronikeinheit durch einen Rastmechanismus mit einer elastisch auslenkbaren Rastlasche an der Befestigungseinheit befestigt ist.

Die Befestigungseinheit ist vorzugsweise frei von elektronischen Komponenten. Sie kann jedoch auch Träger von Sensoren sein und/oder ein elektronisch auslesbares Identifikationsmerkmal aufweisen, beispielsweise ein Identifikationsmerkmal, das in einem RFID-Chip der Befestigungseinheit abgelegt ist und durch einen Sensor der Elektronikeinheit auslesbar ist. Hierdurch kann protokolliert werden, mit welcher Befestigungseinheit die Elektronikeinheit verbunden ist.

Besonders bevorzugt ist es, wenn die elektronische Auswerteeinheit eine Anlageseite aufweist, die im angebrachten Zustand der Auswerteeinheit in Richtung des Gehäuses weist, wobei an der Anlageseite mindestens zwei zueinander parallele Anlagerippen zur Anlage am Gehäuse vorgesehen sind.

Vorzugsweise handelt es sich um genau zwei oder drei zueinander parallele Anlagerippen, wobei die einzelnen Anlagerippen auch unterbrochen sein können und aus zueinander fluchtenden Rippensegmenten bestehen können. Die Rippen bilden erhabene Bereiche zur Anlage am Gehäuse. Die Rippen weisen vorzugsweise eine Länge zwischen 10 mm und 30 mm auf. Die Breite der Rippen beträgt vorzugsweise 5 mm oder weniger, insbesondere vorzugsweise 3 mm oder weniger. An ihrer zum Gehäuse weisenden Seite sind die Rippen vorzugsweise mit einer ausgerundeten Kante versehen.

Zwischen den mindestens zwei Anlagerippen ist ein zurückgesetzter Zwischenbereich vorgesehen, der die Anlagerippen voneinander trennt. In diesem Zwischenbereich ist die Anlageseite der Auswerteeinheit derart zurückgesetzt, so dass sie nicht am Gehäuse des Spenders anliegt. Der Kontakt zwischen der Anlageseite und dem Gehäuse des Spenders beschränkt sich demnach auf die mindestens zwei Anlagerippen.

Eine Auswerteeinheit mit solchen Anlagerippen gestattet eine sichere Anlage am Gehäuse des Spenders, da die Andruckkraft in definierten Anlagebereichen stattfindet. Zudem gestatten die Anlagerippen die Anlage an Gehäusen unterschiedlicher Maße und insbesondere Speicherkörper mit unterschiedlich gewölbter Außenkontur.

Welche Krümmungsradien solcher Außenkonturen jeweils abgedeckt werden, hängt von der Geometrie der Anlageseite ab. Bevorzugt ist eine Gestaltung, bei der die Anlagerippen einen Abstand voneinander von mindestens 8 mm aufweisen, wobei sich die Anlagerippen vorzugsweise um mindestens 1 mm, vorzugsweise um mindestens 1,5 mm über die Anlagefläche in diesem Zwischenbereich erheben. Zwei Anlagerippen, zwischen denen ein Zwischenbereich von 8 mm vorgesehen ist, der 1 mm zurückgesetzt ist, können an Gehäuseabschnitten mit einem Krümmungsradius größer 10 mm angebracht werden, ohne dass der Gehäuseabschnitt abseits der Anlagerippen in Kontakt mit der Anlageseite gelangt. Mit einem Zwischenbereich von 8 mm Weite, der 1,5 mm zurückgesetzt ist, ist die Anbringung bis zu einem Krümmungsradius von ca. 6 mm möglich.

Eine oder mehrere der Anlagerippen können an einem elastischen Ausleger angebracht sein, insbesondere an einem Ausleger in Form einer an drei Seiten freigeschnittenen Kunststoffzunge, die ein federndes Verhalten orthogonal zur Anlageseite ermöglicht. Es kann auch vorgesehen sein, dass eine oder mehrere, nicht aber alle Anlagerippen derartig auslenkbar sind, während mindestens eine Anlagerippe fest und nicht auslenkbar an der Anlageseite der Auswerteeinheit vorgesehen ist. Eine mögliche Konstellation sieht beispielsweise drei Anlagerippen vor, von denen eine oder zwei an jeweils einem elastischen Ausleger angebracht sind, während die dritte Anlagerippe ortsfest an der Anlageseite vorgesehen ist. Auch eine Rippengestaltung mit zwei Anlagerippen, die beide auslenkbar sind, kann zweckmäßig sein.

Die Gestaltung mit Anlagerippen kann sich insbesondere auch als positiv im Kontext der magnetischen Anbringung der Auswerteeinheit am Gehäuse auswirken. Durch die Erstreckungsrichtung der Anlagerippen ist eine Verschieberichtung definiert, in Richtung derer die Auswerteeinheit gegenüber dem Gehäuse verschoben werden kann, um eine Trennung der Auswerteeinheit vom Gehäuse herbeizuführen.

Hierin wird neben dem Spender mitsamt der Auswerteeinheit in seiner Gesamtheit auch die isolierte Auswerteeinheit beschrieben, die zur lösbaren Anbringung an einem Spender in oben beschriebener Weise zur Erfassung der Nutzung des Spenders ausgebildet ist, insbesondere durch Verwendung eines Mikrofons und/oder eines Bewegungssensors.

Um in der oben beschriebenen Weise am Gehäuse eines Spenders angebracht zu werden, ist die Auswerteeinheit gemäß einer ersten Gestaltung im Bereich einer Anlageseite magnetisch oder magnetisierbar ausgebildet. Insbesondere kann mindestens ein Permanentmagnet an der Anlageseite vorgesehen sein, insbesondere ein Neodym-Magnet.

Gemäß einer zweiten Gestaltung weist die Auswerteeinheit mindestens zwei und vorzugsweise mindestens drei elastisch auslenkbare Haltearme auf, die das Gehäuse, insbesondere einen Speicherkörper des Gehäuses, bestimmungsgemäß umgreifen können und damit einen klemmenden Halt der Auswerteinheit am Gehäuse bewirken können.

Die Auswerteeinheit kann in oben bereits beschriebener Weise modular ausgestaltet sein und eine Befestigungseinheit sowie eine Elektronikeinheit aufweisen. Die Befestigungseinheit ist zur lösbaren Anbringung am Gehäuse ausgebildet und im Bereich der Anlageseite magnetisch oder magnetisierbar ausgebildet. Die Elektronikeinheit ist zur lösbaren Anbringung an der Befestigungseinheit vorgesehen und umfasst vorzugsweise die Gesamtheit der elektrischen/elektronischen Komponenten der Auswerteeinheit.

Vorzugsweise kann die Auswerteeinheit mehrere wechselbare Befestigungseinheiten umfassen, die an unterschiedliche Arten von Spendergehäusen angepasst sind und die wahlweise mit der Elektronikeinheit gekoppelt werden können.

### KURZBESCHREIBUNG DER ZEICHNUNGEN

Weitere Vorteile und Aspekte der Erfindung ergeben sich aus den Ansprüchen und aus der nachfolgenden Beschreibung von bevorzugten Ausführungsbeispielen, die nachfolgend anhand der Figuren erläutert sind.
Fig. 1 und 2 zeigen exemplarisch einen Flüssigkeitsspender mit einer Auswerteeinheit.
Fig.3 bis 5 verdeutlichen ein erste, erfindungsgemäße Konfiguration der Ausgestaltung einer magnetischen Verbindung zwischen der Auswerteeinheit und einem Gehäuse des Flüssigkeitsspenders.
Fig. 6 bis 8 verdeutlichen eine zweite, nicht beanspruchte Konfiguration der Ausgestaltung einer magnetischen Verbindung zwischen der Auswerteeinheit und einem Gehäuse des Flüssigkeitsspenders.
Fig. 9 bis 11 verdeutlichen eine nicht beanspruchte Ausgestaltung einer Verbindung zwischen der Auswerteeinheit und einem Gehäuse des Flüssigkeitsspenders durch an der Auswerteeinheit vorgesehene Haltearme.
Fig. 12 bis 15 zeigen eine mögliche modulare Ausgestaltung der Auswerteeinheit mit einer Befestigungseinheit und einer Elektronikeinheitsowie Kopplungsmittel zur Kopplung der Elektronikeinheit an die Befestigungseinheit.
Fig. 16 bis 18 verdeutlichen eine erste Konfiguration von Anlagerippen an einer Anlageseite der Auswerteeinheit.
Fig. 19 bis 21 verdeutlichen eine erste Konfiguration von Anlagerippen an einer Anlageseite der Auswerteeinheit.

### DETAILLIERTE BESCHREIBUNG DER AUSFÜHRUNGSBEISPIELE

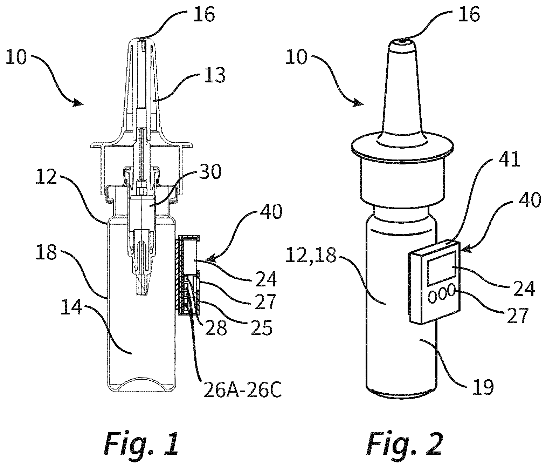

Fig. 1 und 2 zeigen einen Spender 10, der zum Austrag pharmazeutischer Flüssigkeiten ausgebildet ist. Der Flüssigkeitsspender 10 ist exemplarisch als Nasalspender ausgebildet und verfügt über ein Applikatorteil mit einem länglichen Nasalapplikator 13, an dessen distalem Ende eine Austragöffnung 16 vorgesehen ist. Der konkrete Spendertyp ist jedoch nur als Beispiel zu verstehen. Die Erfindung umfasst ebenso auch andere Spender für pharmazeutische oder kosmetische Medien und insbesondere Flüssigkeiten, insbesondere pharmazeutische Spender, die zur oralen oder topischen Abgabe von Medikamenten ausgebildet sind.

Durch Niederdrücken des Applikatorteils gegenüber einem Flüssigkeitsspeicher 14 bzw. dem den Flüssigkeitsspeicher bildenden Speicherkörper 18 kann eine Pumpeinrichtung 30 betätigt werden, die Flüssigkeit aus dem Flüssigkeitsspeicher 14 zur Austragöffnung 16 fördert.

Bei einem erfindungsgemäßen Spender 10 ist vorgesehen, dass die Nutzung des Spenders 10 durch den Anwender elektronisch erfasst wird. Hierfür ist eine elektronische Auswerteeinheit 40 vorgesehen. Diese umfasst im vorliegenden Beispiel eine Batterie 25, eine Anzeigeeinrichtung 24, eine Platine mit einem Prozessor 28, Bedienelemente 27 sowie verschiedene Sensoren 26A bis 26C.

Die Sensoren 26A-26C können insbesondere einen Bewegungssensor umfassen, der die Ausrichtung des Spenders und/oder Beschleunigungen erfasst. Weiterhin können die Sensoren 26A-26C ein Mikrofon umfassen, welches Geräusche erfasst, die bei der Handhabung des Spenders 10 auftreten. Die Auswertung der erfassten Daten erfolgt entweder direkt mittels des Prozessors 28 oder ganz oder teilweise auf einem externen System, welches über eine Funkschnittstelle mit der Auswerteeinheit 40 verbunden ist.

Exemplarisch kann der Prozessor 28 dafür ausgebildet sein, im Falle von mittels des Bewegungssensors erfassten Bewegungen des Spenders 10 das Mikrofon zu aktivieren. Mittels des Mikrofons können Geräusche erfasst werden, insbesondere durch das Spendergehäuse 12 übertragene Geräusche. Insbesondere gehen die verschiedenen Phasen eines Pumpvorgangs, also das Ausbringen von Flüssigkeit aus einer Pumpkammer und das erneute Ansaugen von Flüssigkeit aus dem Medienspeicher 14 in die Pumpkammer mit charakteristischen Geräuschen einher, die gut voneinander unterscheidbar sind.

Die Auswerteeinheit 40 ist an einer Mantelfläche 19 des Speicherkörpers 18 des Spendergehäuses 12 angebracht. Der Speicherkörper 18 weist eine zylindrische Grundform auf, vorliegend eine kreiszylindrische Grundform.

Die Auswerteeinheit 40 umgibt den Speicherkörper 18 nicht, sondern ist rucksackähnlich an einer Seite vorgesehen, so dass sie dem Spender eine asymmetrische Gesamtform verleiht. Die Auswerteeinheit bzw. das den Hauptbestandteil bildende Elektronikgehäuse41 überspannen nureinen vergleichsweise geringen Teil der Mantelfläche, in Umfangsrichtung vorliegend in etwa 90° oder ein Viertel des Umfangs.

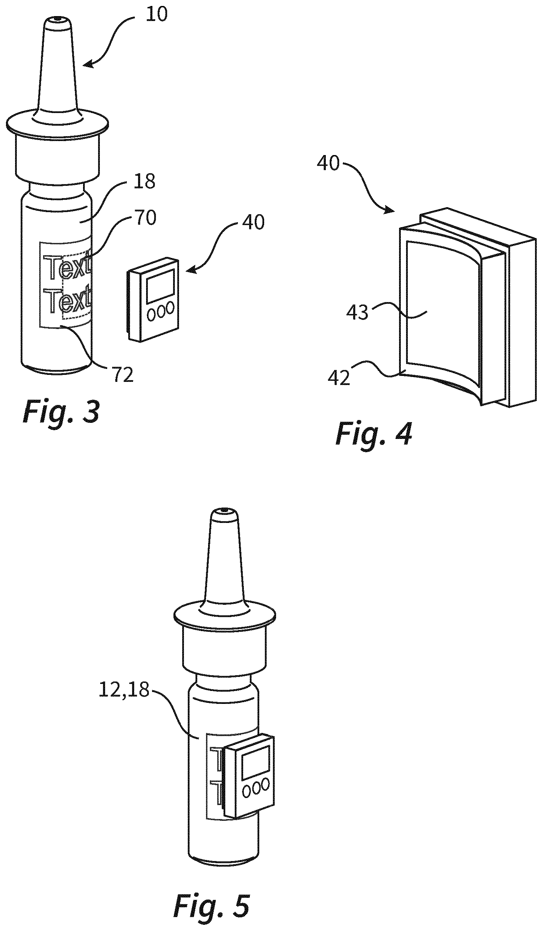

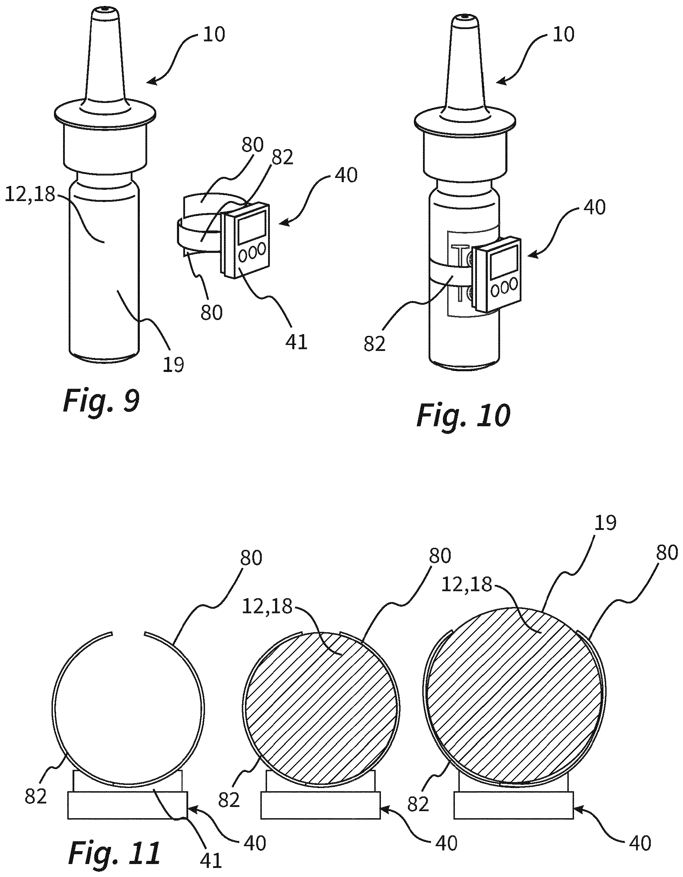

Zur Anbringung der Auswerteeinheit 40 am Speicherkörper 18 werden zwei Befestigungsprinzipien vorgeschlagen, die anhand der Fig. 3 bis 8 bzw. anhand der Fig. 9 bis 11 vorgestellt werden.

Bei der Gestaltung der Fig. 3 bis 8 ist vorgesehen, dass die Auswerteinheit 40 magnetisch am Speicherkörper 18 befestigt ist.

Fig. 3 zeigt den Speicherkörper 18 des Spenders 10, der mit einem Etikett 72 versehen ist, welches beispielsweise die Produktbezeichnung oder Produktdetails enthalten kann oder aber auch Handhabungshinweise zur Anbringung der Auswerteeinheit 40. Erfindungsgemäß ist in das Etikett 72 integriert oder vom Etikett 72 überdeckt eine magnetische oder magnetisierbare Haltefläche 70 vorgesehen. Insbesondere kann es sich dabei um einen rechteckigen metallischen Blechabschnitt handeln.

Wie in Fig. 4 dargestellt ist, ist korrespondierend hierzu an einer Anlageseite 42 der Auswerteeinheit 40 ein Permanentmagnet 43 vorgesehen. Die Form und Größe der Haltefläche 70 und des Permanentmagneten 43 sind vorzugsweise derart aufeinander abgestimmt, dass die Auswerteinheit 40 in einer definierten Position am Speicherkörper 18 hält. Der so verbundene Zustand ist in Fig. 5 dargestellt.

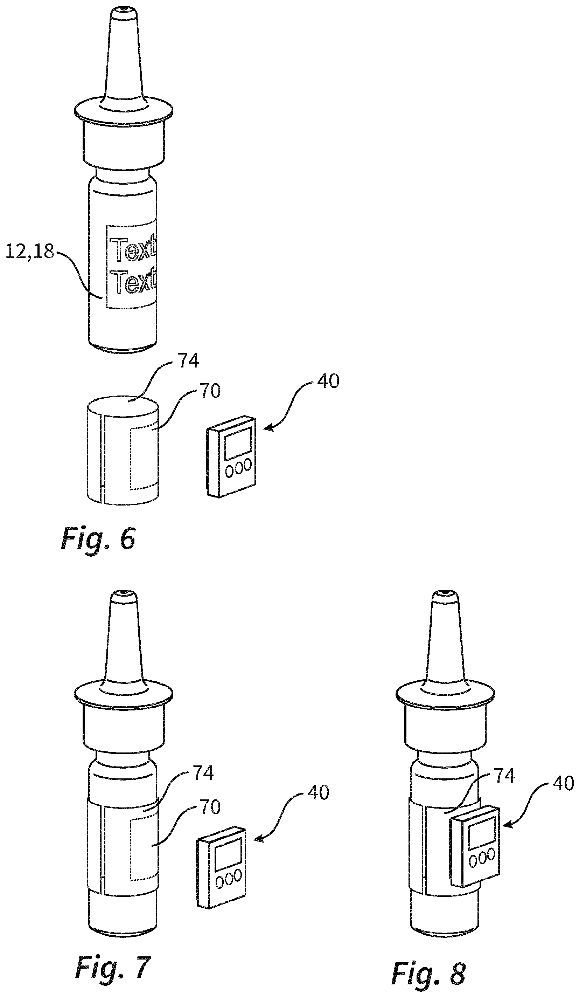

Fig. 6 zeigt eine Gestaltung, bei der eine Haltehülse 74vorgesehen ist, die vollständig oder zumindest im Bereich einer Haltefläche 70 magnetisierbar ausgestaltet ist. Diese Haltehülse 74 ist als geschlitzte Hülse ausgebildet und kann in der in Fig. 7 verdeutlichten Weise auf den Speicherkörper 18 aufgeschoben werden, wobei der Innendurchmesser der Haltehülse 74 an den Außendurchmesser des Speicherkörpers 18 derart angepasst ist, dass die Haltehülse 74 nach dem Aufschieben auf den Speicherkörper 18 elastisch aufgeweitet ist und hierdurch klemmend am Speicherkörper 18 hält.

Die Haltefläche 70 als Teil der Haltehülse gestattet in der in Fig. 8 verdeutlichten Weise die magnetische Anbringung der Auswerteeinheit 40.

Die Fig. 9 bis 11 zeigen eine andere Form der Befestigung einer Auswerteeinheit 40 am Speicherkörper 18. Wie anhand der Fig. 9 ersichtlich ist, ist bei dieser Gestaltung vorgesehen, dass am Gehäuse der Auswerteeinheit insgesamt drei Haltearme 80, 82 vorgesehen sind, die jeweils eine gekrümmte Form aufweisen und gemeinsam einen Bereich zur Aufnahme des Speicherkörpers 18 umgeben. Die Haltearme 80, 82 sind mit ihrer Wurzel fest am Elektronikgehäuse 41 angebracht.

Der obere und der untere Haltearm 80 erstrecken sich vom Gehäuse der Auswerteeinheit 40 gegen den Uhrzeigersinn. Der dazwischenliegende Haltearm 82 erstreckt sich um das Gehäuse der Auswerteeinheit 40 aus im Uhrzeigersinn.

Im nicht angebrachten Zustand der Fig. 9 sind die Haltearme 80, 82 nicht elastisch ausgelenkt. Wird jedoch der Speicherkörper 18 in den von den Haltearmen umgebenen Bereich einschoben, so werden die Haltearme hierdurch leicht auseinander gedrückt, so dass sie klemmend an der Außenseite des Speicherkörpers anliegen und hierdurch eine sichere Kopplung schaffen. Der montierte Zustand ist in Fig. 10 dargestellt.

Fig. 11 verdeutlicht, wie die Auswerteeinheit an Gehäusen 12 unterschiedlichen Durchmessers angebracht werden kann. Die linke Darstellung der Fig. 11 zeigt die Auswerteeinheit 40 im nicht genutzten Zustand. Die mittlere Darstellung der Fig. 11 zeigt die Auswerteeinheit 40 nach Ankopplung an ein Gehäuse 12, dessen Außendurchmesser nur geringfügig größer als der Ruhezustand der Haltearme 80, 82 ist. Die Haltearme 80, 82 sind hierdurch elastisch nach außen ausgelenkt und klemmen den Speicherkörper 18 daher ein. Die Darstellung rechts in Fig. 11 zeigt die Verwendung der Auswerteeinheit 40 mit einem bedeutend größeren Gehäuse 12. Die Haltearme 80, 82 sind hier deutlich weiter aufgeweitet. Hierdurch bedingt liegen sie nicht über ihre gesamte Länge an der Außenseite des Gehäuses 12 an. Da jedoch die Auslenkung und hiermit einhergehend die Rückstellkraft erheblich größer ist, wird dennoch ein sicherer Halt auch an diesem Gehäuse 12 erzielt.

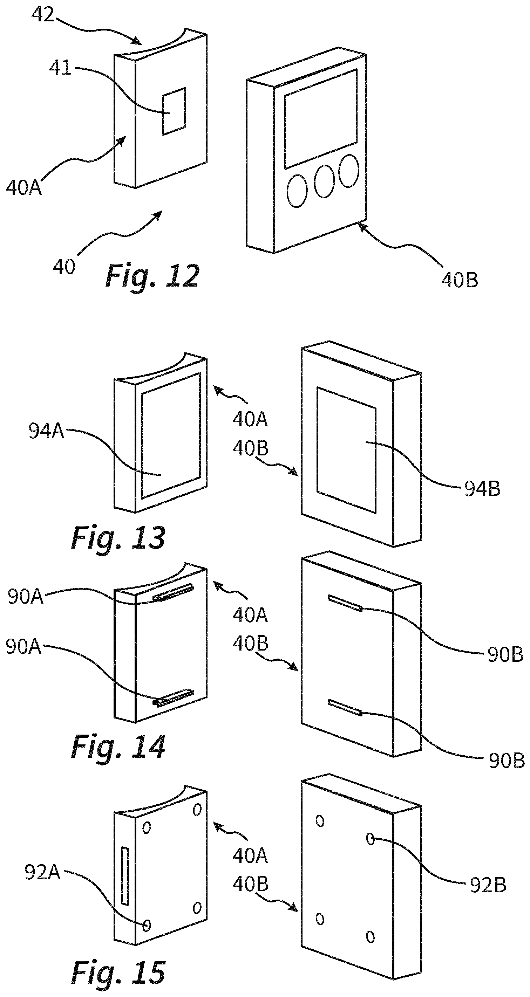

Die Fig. 12 bis 15 verdeutlichen die Möglichkeit, die Auswerteeinheit 40 modular zu gestalten. In nicht näher dargestellter Weise kann die Anlageseite 12 der Auswerteeinheit 40 entsprechend den Fig. 3 bis 8 magnetisch oder magnetisierbar ausgestaltet sein oder mit Haltearmen entsprechend der Fig. 9 bis 11 versehen sein.

Wie anhand der Fig. 12 verdeutlicht, untergliedert sich die Auswerteeinheit 40 in solchen Fällen in eine Befestigungseinheit 40A, an der die Anlageseite 42 vorgesehen ist, sowie in eine Elektronikeinheit 40B, die die überwiegende Zahl an elektrischen/elektronischen Komponenten aufweist, vorzugsweise die Gesamtheit der elektronischen Komponenten. Es kann allerdings vorgesehen sein, dass die Befestigungseinheit 40A über ein elektronisches Identifikationsmittel wie einen RFID-Chip 41 verfügt, damit die Elektronikeinheit 40B erkennen kann, ob sie mit einer Befestigungseinheit 40A verbunden ist bzw. mit welcher Befestigungseinheit 40A sie verbunden ist.

Die Teileinheiten 40A, 40B sind vorzugsweise werkzeuglos miteinander koppelbar. Bei der Gestaltung der Fig. 13 sind die beiden Teileinheiten mit magnetischen oder magnetisierbaren Kopplungsflächen 94A, 94B verbunden. Bei einer solchen Gestaltung ist es möglich, einen gemeinsamen Magneten an der Befestigungseinheit 40A vorzusehen, der der Kopplung an das Gehäuse 12 sowie der Kopplung mit der Elektronikeinheit 40B dient.

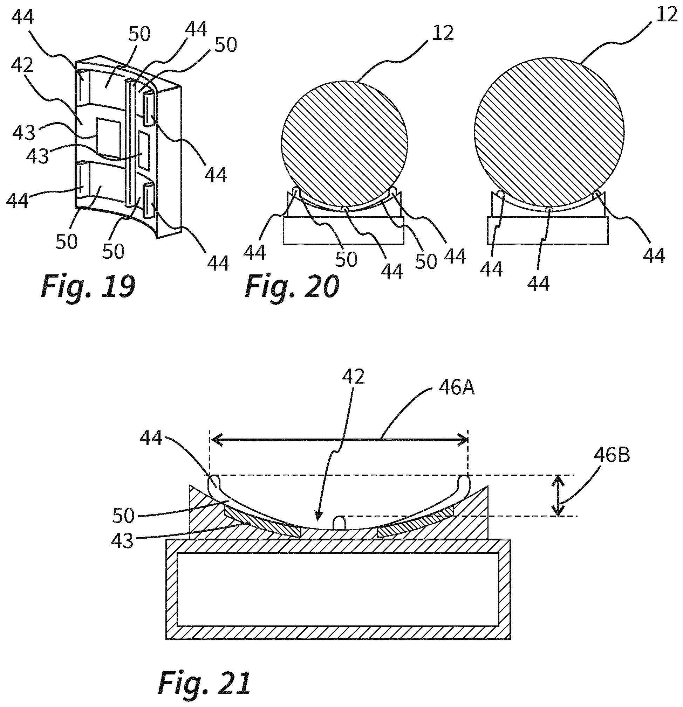

Bei der Gestaltung der Fig. 13 sind Rastlaschen 90A an der Befestigungseinheit 40A vorgesehen, die in Rastöffnungen 90B an der Elektronikeinheit 40B verrasten. Vorzugsweise sind die Rastlaschen 90A abweichend von der Gestaltung der Fig. 20 manuell auslenkbar, um die Verbindung wieder trennen zu können. Es kann jedoch auch vorgesehen sein, dass die Verbindung der Elektronikeinheit 40B mit der Befestigungseinheit 40A bestimmungsgemäß unlösbar ist, so dass eine entsprechende Kopplung nur einmalig möglich ist, insbesondere bei einer bereits im Zuge der Herstellung stattfindenden Festlegung auf eine bestimmte Befestigungseinheit 40A.

Bei der Gestaltung der Fig. 14 sind Schraublöcher 92A, 92B an der Elektronikeinheit 40B und der Befestigungseinheit 40A vorgesehen, durch die hindurch die beiden Teileinheiten miteinander verschraubt werden können. Diese Art der Verbindung ist üblicherweise nicht dafür vorgesehen, vom Endanwender gelöst zu werden, sondern dient dem Zweck, im Zuge der Herstellung eine einheitliche Elektronikeinheit 40B dauerhaft mit einer Befestigungseinheit 40A verbinden zu können.

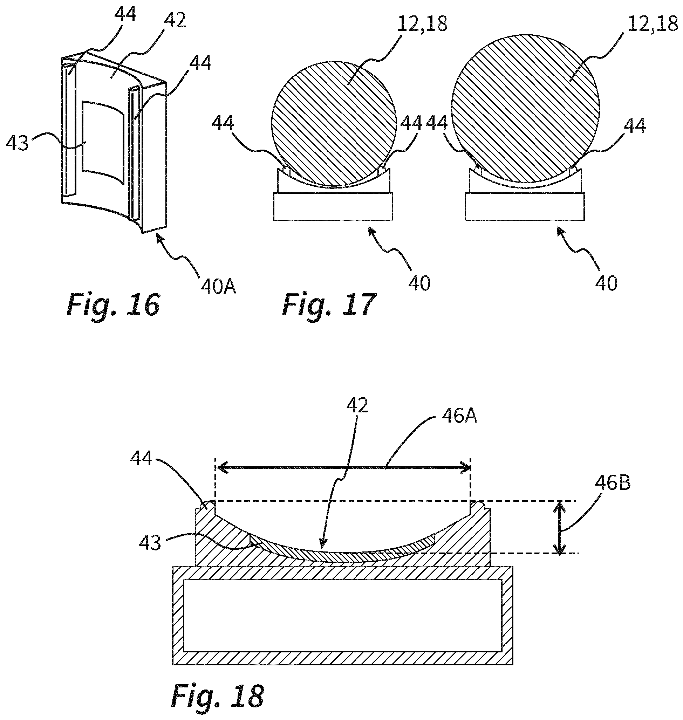

Die Fig. 16 bis 21 verdeutlichen, dass es von Vorteil sein kann, wenn die Auswertungseinheit 40 bzw. ihre Befestigungseinheit 40A mit Anlagerippen 44 versehen ist. Die zur Anlage am Gehäuse 12 bzw. der Haltehülse 74 vorgesehen sind.

Die Fig. 16 bis 21 zeigen eine erste Konfiguration von Anlagerippen 44. In Fig. 16 ist die Rückseite der Auswerteeinheit 40 dargestellt. Diese Rückseite bildet eine Anlageseite 42, wobei zwei parallele Anlagerippen 44 zueinander ortsfest an der Anlageseite 42 vorgesehen sind. Zwischen den beiden Anlagerippen 44 ist ein zurückgesetzter Zwischenbereich vorgesehen, wobei hier ein Permanentmagnet 43 befestigt ist. Wie sich anhand der Fig. 17 ersehen lässt, ermöglichen es die Anlagerippen 44, an Spendergehäusen 12 unterschiedlicher Krümmungsradien anzuliegen. Die gleiche Auswerteeinheit 40 bzw. die gleiche Befestigungseinheit 40A der Auswerteeinheit 40 kann also für unterschiedliche Spender 10 gleichermaßen gut verwendet werden.

Fig. 18 verdeutlicht nochmals die Geometrie der Anlageseite 42. Es ist zu ersehen, dass diese eine grundsätzlich gewölbte konkave Form aufweist und dass die beiden Anlagerippen 44 sich über diese gewölbte konkave Form erheben. Der Abstand 46A entspricht der Breite des Zwischenbereichs. Dieser Abstand ist zur Erfüllung der Rippenfunktion geringer als der Durchmesser des Speicherkörpers 18. Vorzugsweise beträgt er maximal 80% des Durchmessers, bezogen auf den horizontalen Durchmesser in der Ausrichtung der Fig. 17.

Der Abstand 46B zeigt an, wieweit der Zwischenbereich maximal gegenüber den Anlagerippen 44 zurückgesetzt ist. Da im Zwischenbereich auch der Permanentmagnet 43 angeordnet ist, ist diese aufgrund der Anlagerippen 44 etwas von der Außenseite des Spendergehäuses 12 beabstandet. Bei Wahl von ausreichend starken Magneten, insbesondere Neodym-Magneten, steht dieser Abstand einer ausreichenden Haltekraft jedoch nicht entgegen.

Die Fig. 19 bis 21 zeigen eine zweite Konfiguration von Anlagerippen 44. Wie anhand der Fig. 19 zu ersehen ist, ist die Anlageseite 42 hier mit insgesamt drei Anlagerippen 44 versehen. Eine zentrische Anlagerippe 44 ist fest mit dem Gehäuse der Auswerteeinheit 40 verbunden und gegenüber diesem nicht in relevantem Maße auslenkbar. Zwei seitliche Anlagerippen 44 sind dagegen aus Auslegern 50 vorgesehen, wobei beide seitliche Anlagerippen 44 jeweils aus zwei miteinander fluchtenden Rippensegmenten bestehen, die ihrerseits jeweils an einem Ausleger 50 vorgesehen sind. Die beiden äußeren Anlagerippen können daher federnd ausgelenkt werden.

Die Ausleger 50 gestatten es, die außenliegenden Anlagerippen 44 gegen die Federkraft der Ausleger 50 relativ zueinander zu verlagern. Hierdurch kann trotz insgesamt dreier Anlagerippen erreicht werden, dass alle Anlagerippen bei Verwendung der Auswerteeinheit 40 mit unterschiedlichen Spendergehäusen 12 bzw. Speicherkörpern 18 jeweils am Spendergehäuse 12 anliegen. Bei Spendergehäusen 12/Speicherkörpern 18 mit größeren Durchmessern werden die Ausleger 50 elastisch ausgelenkt.

## Patentansprüche

1. Spender (10) zum Austrag pharmazeutischer oder kosmetischer Medien mit den folgenden Merkmalen:
a. der Spender (10) weist ein Gehäuse (12) auf, und
b. der Spender (10) weist einen Medienspeicher (14) auf, und
c. der Spender (10) weist eine Austragöffnung (16) auf, durch die Medium aus dem Medienspeicher (14) in eine umgebende Atmosphäre abgegeben werden kann, und
d. der Spender (10) weist eine elektronische Auswerteeinheit (40) zur Erfassung der Nutzung des Spenders auf, und
e. die elektronische Auswerteeinheit (40) ist zur lösbaren Anbringung am Gehäuse (12) des Spenders (10) ausgebildet, und
f. an der Außenseite des Gehäuses (12), insbesondere an einem Speicherkörper (18) des Gehäuses (12), ist eine magnetische oder magnetisierbare Haltefläche (70) vorgesehen, und
g. eine Anlageseite (42) der Auswerteeinheit (40) ist hierzu korrespondierend magnetisch oder magnetisierbar ausgebildet, so dass die Auswerteeinheit (40) magnetisch am Gehäuse (12) befestigt werden kann,
**gekennzeichnet durch** das folgende weitere Merkmal:
h. die magnetische oder magnetisierbare Haltefläche (70) ist durch ein beschriftetes Etikett (72) überdeckt oder in ein beschriftetes Etikett integriert.

2. Spender (10) nach Anspruch 1 mit dem folgenden weiteren Merkmal:
a. die elektronische Auswerteeinheit (40) ist zur lösbaren Anbringung an einer Mantelfläche (19) des Speicherkörper (18) des Gehäuses vorgesehen, wobei die Außenseite am Speicherkörper (18) mit der magnetischen oder magnetisierbaren Haltefläche (70) versehen ist und wobei die Mantelfläche (19) vorzugsweise eine kreiszylindrische Mantelfläche (19) ist.

3. Spender (10) nach Anspruch 2 mit dem folgenden weiteren Merkmal:
a. die Außenseite des Speicherkörpers (18) und die magnetische oder magnetisierbare Haltefläche (70) weisen eine gewölbte Formgebung auf.

4. Spender (10) nach Anspruch 2 oder 3 mit den folgenden weiteren Merkmalen:
a. die Auswerteinheit (40) weist ein Elektronikgehäuse (41) auf, innerhalb dessen die elektronischen Komponenten der Auswerteinheit (40) angeordnet sind, und
b. das Elektronikgehäuse (41) überdeckt in Umfangsrichtung höchstens 50% der Mantelfläche des Speicherkörpers, vorzugsweise höchstens 40%.

5. Spender (10) nach einem der vorstehenden Ansprüche mit den folgenden weiteren Merkmalen:
a. der Speicherkörper (18) ist primär aus Kunststoff gefertigt, und
b. die magnetische oder magnetisierbare Haltefläche (70) ist außenseitig an einer Kunststoffwandung des Speicherkörpers (18) fest angebracht.

6. Spender (10) nach einem der vorstehenden Ansprüche mit mindestens einem der folgenden weiteren Merkmale:
a. die Beschriftung umfasst eine Produktbezeichnung und/oder eine Produktbeschreibung, und/oder
b. die Beschriftung umfasst eine Handhabungsanleitung betreffend die Anbringung der Auswerteeinheit.

7. Spender (10) nach Anspruch 1 oder 6 mit dem folgenden weiteren Merkmal:
a. die magnetische oder magnetisierbare Haltefläche (70) wird durch eine am Gehäuse klemmend befestigte Haltehülse (74) oder Halteklemme gebildet.

8. Spender (10) nach einem der vorstehenden Ansprüche mit dem folgenden weiteren Merkmal:
a. die magnetische oder magnetisierbare Haltefläche (70) ist klebend am Gehäuse befestigt.

9. Spender (10) nach einem der vorstehenden Ansprüche mit den folgenden weiteren Merkmalen:
a. die Auswerteeinheit (40) umfasst eine Befestigungseinheit (40A) und eine Elektronikeinheit (40B), und
b. die Befestigungseinheit (40A) ist zur lösbaren Anbringung am Gehäuse (12) ausgebildet, und
c. die Elektronikeinheit (40B) ist zur lösbaren Anbringung an der Befestigungseinheit (40A) vorgesehen.

10. Spender (10) nach Anspruch 9 mit mindestens einem der folgenden zusätzlichen Merkmale:
a. die Befestigungseinheit (40A) ist zur werkzeuglos lösbaren Anbringung am Gehäuse (12) ausgebildet, und/oder
b. die Elektronikeinheit (40B) ist magnetisch an der Befestigungseinheit (40A) befestigt, und/oder
c. die Elektronikeinheit (40B) ist durch einen Rastmechanismus mit einer elastisch auslenkbaren Rastlasche (90) an der Befestigungseinheit (40A) befestigt, und/oder
d. die Befestigungseinheit (40A) weist ein Identifikationsmerkmal auf, insbesondere abgelegt in einem RFID-Chip der Befestigungseinheit, welcher durch einen Sensor (26B) der Elektronikeinheit (40B) auslesbar ist.

11. Spender (10) nach einem der vorstehenden Ansprüche mit den folgenden Merkmalen:
a. die elektronische Auswerteeinheit (40) weist eine Anlageseite (42) auf, die im angebrachten Zustand der Auswerteeinheit (40) in Richtung des Gehäuses (12) weist, und
b. die elektronische Auswerteeinheit (40) weist an der Anlageseite (42) zwei zueinander parallele Anlagerippen (44) zur Anlage am Gehäuse (12) auf, insbesondere zur Anlage an einem Speicherkörper (18) des Medienspeichers (14).

12. Spender (10) nach Anspruch 11 mit mindestens einem der folgenden weiteren Merkmale:
a. die Rippen (44) weisen einen Abstand (46A) voneinander von mindestens 8 mm auf, und/oder
b. die Rippen (44) erheben sich gegenüber einem zwischen ihnen liegenden Zwischenbereich (48) um einen Abstand (46B) von mindestens 1,5 mm und/oder
c. mindestens eine Rippe (44) ist an einem elastischen Ausleger (50) angebracht, und/oder
d. mindestens eine Rippe (44) wird durch mehrere zueinander fluchtende Rippensegmente gebildet, die vorzugsweise jeweils an einem elastischen Ausleger (50) vorgesehen sind.

13. Spender (10) nach einem der vorstehenden Ansprüche mit mindestens einem der folgenden weiteren Merkmale:
a. die Auswerteeinheit (40) umfasst mindestens einen Sensor (26A, 26B, 26C), insbesondere einen Bewegungssensor (26A) und/oder ein Mikrofon (26C), und/oder
b. die Auswerteeinheit (40) umfasst eine Ausgabeeinrichtung (24), insbesondere in Form mindestens einer LED, eines Displays (24), eines Vibrators und/oder eines Lautsprechers,
c. die Auswerteeinheit umfasst ein Funkmodul (22), insbesondere ein Bluetooth- und/oder WIFI-Funkmodul (22), und/oder
d. die Auswerteeinheit umfasst eine Energiequelle (25), insbesondere in Form einer Batterie.

14. Spender (10) nach einem der vorstehenden Ansprüche mit dem folgenden weiteren Merkmal:
a. der Spender (10) ist als Flüssigkeitsspender (10) ausgebildet, wobei der Medienspeicher (14) als Flüssigkeitsspeicher (14) ausgebildet ist,
insbesondere mit mindestens einem der folgenden zusätzlichen Merkmale:
b. der Spender ist als Quetschflaschenspender ausgebildet und weist einen elastisch verformbaren Speicherkörper des Flüssigkeitsspeichers auf, durch dessen manuelle Komprimierung Flüssigkeit ausgetragen werden kann, oder
c. der Spender (10) ist als Pumpspender ausgebildet, der über eine manuell betätigbare Pumpeinrichtung (30) verfügt, die Flüssigkeit aus dem Flüssigkeitsspeicher (14) zur Austragöffnung (16) fördert, und/oder
d. der Spender (10) ist als Sprühspender ausgebildet, und/oder
e. der Spender ist als Inhalator ausgebildet, insbesondere als Spender mit einem zur Abgabe einer Dosis der Flüssigkeit niederdrückbarem Container,
f. der Spender ist als Tropfenspender ausgebildet, insbesondere mit einer die Austragöffnung umgebenden Tropfenbildungsfläche, und/oder
g. der Spender ist als Nasalspender ausgebildet, insbesondere mit einem langgestreckten Nasalapplikator, an dessen distalem Ende die Austragöffnung vorgesehen ist.

## Claims

1. Dispenser (10) for the discharge of pharmaceutical or cosmetic media, with the following features:
a. the dispenser (10) has a housing (12), and
b. the dispenser (10) has a medium reservoir (14), and
c. the dispenser (10) has a discharge opening (16), through which medium can be discharged from the medium reservoir (14) into a surrounding atmosphere, and
d. the dispenser (10) has an electronic evaluation unit (40) for detecting the use of the dispenser, and
e. the electronic evaluation unit (40) is designed for releasable attachment to the housing (12) of the dispenser (10), and
f. a magnetic or magnetizable holding surface (70) is provided on the outer side of the housing (12), in particular on a reservoir body (18) of the housing (12), and
g. a contact side (42) of the evaluation unit (40) is of corresponding magnetic or magnetizable design to this end, such that the evaluation unit (40) can be magnetically fastened to the housing (12),
**characterized by** the following further feature:
h. the magnetic or magnetizable holding surface (70) is covered by an label (72) with an inscription or integrated into an label with an inscription.

2. Dispenser (10) according to Claim 1, with the following further feature:
a. the electronic evaluation unit (40) is provided for releasable attachment to a lateral surface (19) of the reservoir body (18) of the housing, wherein the outer side on the reservoir body (18) is provided with the magnetic or magnetizable holding surface (70), and wherein the lateral surface (19) is preferably a circular-cylindrical lateral surface (19).

3. Dispenser (10) according to Claim 2, with the following further feature:
a. the outer side of the reservoir body (18) and the magnetic or magnetizable holding surface (70) have a curved shape.

4. Dispenser (10) according to Claim 2 or 3, with the following further features:
a. the evaluation unit (40) has an electronics housing (41), within which the electronic components of the evaluation unit (40) are arranged, and
b. the electronics housing (41) covers at most 50% of the lateral surface of the reservoir body, preferably at most 40%, in the circumferential direction.

5. Dispenser (10) according to one of the preceding claims, with the following further features:
a. the reservoir body (18) is primarily manufactured from plastic, and
b. the magnetic or magnetizable holding surface (70) is fixedly attached on the outside to a plastic wall of the reservoir body (18).

6. Dispenser (10) according to one of the preceding claims, with at least one of the following further features:
a. the inscription comprises a product designation and/or a product description, and/or
b. the inscription comprises handling instructions relating to the attachment of the evaluation unit.

7. Dispenser (10) according to Claim 1 or 6, with the following further feature:
a. the magnetic or magnetizable holding surface (70) is formed by a holding sleeve (74) or holding clamp fastened in a clamping manner to the housing.

8. Dispenser (10) according to one of the preceding claims, with the following further feature:
a. the magnetic or magnetizable holding surface (70) is adhesively fastened to the housing.

9. Dispenser (10) according to one of the preceding claims, with the following further features:
a. the evaluation unit (40) comprises a fastening unit (40A) and an electronics unit (40B), and
b. the fastening unit (40A) is designed for releasable attachment to the housing (12), and
c. the electronics unit (40B) is provided for releasable attachment to the fastening unit (40A).

10. Dispenser (10) according to Claim 9, with at least one of the following additional features:
a. the fastening unit (40A) is designed for the releasable attachment to the housing (12) without tools, and/or
b. the electronics unit (40B) is fastened magnetically to the fastening unit (40A), and/or
c. the electronics unit (40B) is fastened to the fastening unit (40A) by means of a latching mechanism having an elastically deflectable latching lug (90), and/or
d. the fastening unit (40A) has an identification feature, in particular stored in an RFID chip of the fastening unit, which can be read out by a sensor (26B) of the electronics unit (40B).

11. Dispenser (10) according to one of the preceding claims, with the following features:
a. the electronic evaluation unit (40) has a contact side (42) which, in the attached state of the evaluation unit (40), points in the direction of the housing (12), and
b. the electronic evaluation unit (40) has, on the contact side (42), two mutually parallel contact ribs (44) for bearing against the housing (12), in particular for bearing against a reservoir body (18) of the medium reservoir (14).

12. Dispenser (10) according to Claim 11, with at least one of the following further features:
a. the ribs (44) are at a distance (46A) from one another of at least 8 mm, and/or
b. the ribs (44) rise up with respect to an intermediate region (48) lying between them by a distance (46B) of at least 1,5 mm, and/or
c. at least one rib (44) is attached to an elastic bracket (50), and/or
d. at least one rib (44) is formed by a number of rib segments which are aligned with one another and are preferably each provided on an elastic bracket (50).

13. Dispenser (10) according to one of the preceding claims, with at least one of the following further features:
a. the evaluation unit (40) comprises at least one sensor (26A, 26B, 26C), in particular a movement sensor (26A) and/or a microphone (26C), and/or
b. the evaluation unit (40) comprises an output device (24), in particular in the form of at least one LED, a display (24), a vibrator and/or a loudspeaker,
c. the evaluation unit comprises a radio module (22), in particular a Bluetooth and/or Wi-Fi radio module (22), and/or
d. the evaluation unit comprises an energy source (25), in particular in the form of a battery.

14. Dispenser (10) according to one of the preceding claims, with the following further feature:
a. the dispenser (10) is designed as a liquid dispenser (10), wherein the medium reservoir (14) is designed as a liquid reservoir (14),
in particular with at least one of the following additional features:
b. the dispenser is designed as a squeeze bottle dispenser and has an elastically deformable reservoir body of the liquid reservoir, by means of the manual compression of which liquid can be discharged, or
c. the dispenser (10) is designed as a pump dispenser which has a manually actuable pump device (30) which conveys liquid from the liquid reservoir (14) to the discharge opening (16), and/or
d. the dispenser (10) is designed as a spray dispenser, and/or
e. the dispenser is designed as an inhaler, in particular as a dispenser with a container which can be depressed for dispensing a dose of the liquid,
f. the dispenser is designed as a droplet dispenser, in particular with a droplet formation surface surrounding the discharge opening, and/or
g. the dispenser is designed as a nasal dispenser, in particular with an elongate nasal applicator, at the distal end of which the discharge opening is provided.

## Revendications

1. Distributeur (10) pour l'évacuation de milieux pharmaceutiques ou cosmétiques, présentant les caractéristiques suivantes :
a. le distributeur (10) comporte un boîtier (12), et
b. le distributeur (10) comporte un réservoir de milieu (14), et
c. le distributeur (10) présente une ouverture d'évacuation (16) à travers laquelle le milieu peut être déchargé du réservoir de milieu (14) dans une atmosphère environnante, et
d. le distributeur (10) comporte une unité d'évaluation électronique (40) pour détecter l'utilisation du distributeur, et
e. l'unité d'évaluation électronique (40) est conçue pour être fixée de manière amovible au boîtier (12) du distributeur (10), et
f. il est prévu une surface de maintien (70) magnétique ou magnétisable sur la face extérieure du boîtier (12), en particulier sur un corps de réservoir (18) du boîtier (12), et
g. une face de butée (42) de l'unité d'évaluation (40) est à cet effet conçue de manière correspondante sous forme magnétique ou magnétisable, de sorte que l'unité d'évaluation (40) peut être fixée magnétiquement au boîtier (12),
**caractérisé par** l'autre caractéristique suivante :
h. la surface de maintien magnétique ou magnétisable (70) est recouverte d'une étiquette portant une inscription (72) ou intégrée dans une étiquette portant une inscription.

2. Distributeur (10) selon la revendication 1, présentant l'autre caractéristique suivante :
a. l'unité d'évaluation électronique (40) est prévue pour être fixée de manière amovible à une surface extérieure (19) du corps de réservoir (18) du boîtier, la face extérieure du corps de réservoir (18) étant pourvue de la surface de maintien magnétique ou magnétisable (70), la surface extérieure (19) étant de préférence une surface extérieure cylindrique circulaire (19).

3. Distributeur (10) selon la revendication 2, présentant l'autre caractéristique suivante :
a. la face extérieure du corps de réservoir (18) et la surface de maintien magnétique ou magnétisable (70) présentent une forme bombée.

4. Distributeur (10) selon la revendication 2 ou 3, présentant les autres caractéristiques suivantes :
a. l'unité d'évaluation (40) comporte un boîtier électronique (41) dans lequel sont disposés les composants électroniques de l'unité d'évaluation (40), et
b. le boîtier électronique (41) recouvre au maximum 50 % de la surface extérieure du corps de réservoir, de préférence au maximum 40 %, dans la direction circonférentielle.

5. Distributeur (10) selon l'une quelconque des revendications précédentes, présentant les autres caractéristiques suivantes :
a. le corps de réservoir (18) est principalement fabriqué en plastique, et
b. la surface de maintien magnétique ou magnétisable (70) est installée de manière fixe à l'extérieur sur une paroi en plastique du corps de réservoir (18).

6. Distributeur (10) selon l'une quelconque des revendications précédentes, présentant au moins l'une des autres caractéristiques suivantes :
a. l'inscription comprend une désignation de produit et/ou une description de produit, et/ou
b. l'inscription comprend une instruction de manipulation relative à l'installation de l'unité d'évaluation.

7. Distributeur (10) selon la revendication 1 ou 6, présentant l'autre caractéristique suivante :
a. la surface de maintien magnétique ou magnétisable (70) est formée par une douille de maintien (74) ou une pince de maintien fixée par serrage au boîtier.

8. Distributeur (10) selon l'une quelconque des revendications précédentes, présentant l'autre caractéristique suivante :
a. la surface de maintien magnétique ou magnétisable (70) est fixée de manière adhésive au boîtier.

9. Distributeur (10) selon l'une quelconque des revendications précédentes, présentant les autres caractéristiques suivantes :
a. l'unité d'évaluation (40) comprend une unité de fixation (40A) et une unité électronique (40B), et
b. l'unité de fixation (40A) est conçue pour être fixée de manière amovible au boîtier (12), et
c. l'unité électronique (40B) est prévue pour être fixée de manière amovible à l'unité de fixation (40A).

10. Distributeur (10) selon la revendication 9, présentant au moins l'une des caractéristiques supplémentaires suivantes :
a. l'unité de fixation (40A) est conçue pour être fixée de manière amovible au boîtier (12) sans outils, et/ou
b. l'unité électronique (40B) est fixée magnétiquement à l'unité de fixation (40A), et/ou
c. l'unité électronique (40B) est fixée à l'unité de fixation (40A) au moyen d'un mécanisme d'encliquetage présentant une patte d'encliquetage (90) élastiquement déformable, et/ou
d. l'unité de fixation (40A) présente une caractéristique d'identification, en particulier stockée dans une puce RFID de l'unité de fixation, qui peut être lue par un capteur (26B) de l'unité électronique (40B).

11. Distributeur (10) selon l'une des revendications précédentes, présentant les caractéristiques suivantes :
a. l'unité d'évaluation électronique (40) présente une face de butée (42) qui, lorsque l'unité d'évaluation (40) est montée, est orientée en direction du boîtier (12), et
b. l'unité électronique d'évaluation (40) comporte, sur la face de butée (42), deux nervures de butée (44) parallèles l'une à l'autre, destinées à venir en butée contre le boîtier (12), en particulier contre un corps de réservoir (18) du réservoir de milieu (14).

12. Distributeur (10) selon la revendication 11, présentant au moins l'une des autres caractéristiques suivantes :
a. les nervures (44) sont espacées les unes des autres d'une distance (46A) d'au moins 8 mm, et/ou
b. les nervures (44) s'élèvent par rapport à une zone intermédiaire (48) située entre elles d'une distance (46B) d'au moins 1,5 mm et/ou
c. au moins une nervure (44) est fixée à une flèche élastique (50), et/ou
d. au moins une nervure (44) est formée par une pluralité de segments de nervure alignés les uns par rapport aux autres et de préférence respectivement disposés sur une flèche élastique (50).

13. Distributeur (10) selon l'une quelconque des revendications précédentes, présentant au moins l'une des autres caractéristiques suivantes :
a. l'unité d'évaluation (40) comprend au moins un capteur (26A, 26B, 26C), en particulier un capteur de mouvement (26A) et/ou un microphone (26C), et/ou
b. l'unité d'évaluation (40) comprend un dispositif de sortie (24), notamment sous la forme d'au moins une DEL, d'un dispositif d'affichage (24), d'un vibreur et/ou d'un haut-parleur,
c. l'unité d'évaluation comprend un module radio (22), en particulier un module radio Bluetooth et/ou WIFI (22), et/ou
d. l'unité d'évaluation comprend une source d'énergie (25), en particulier sous la forme d'une batterie.

14. Distributeur (10) selon l'une quelconque des revendications précédentes, présentant l'autre caractéristique suivante :
a. le distributeur (10) est conçu sous la forme d'un distributeur de liquide (10), le réservoir de milieu (14) étant conçu sous la forme d'un réservoir de liquide (14),
en particulier avec au moins une des caractéristiques supplémentaires suivantes :
b. le distributeur est conçu sous la forme d'un distributeur à flacon souple et comporte un corps de réservoir élastiquement déformable du réservoir de liquide, par la compression manuelle duquel du liquide peut être expulsé, ou
c. le distributeur (10) est conçu sous la forme d'un distributeur à pompe qui comporte un dispositif de pompage (30) actionnable manuellement qui achemine le liquide du réservoir de liquide (14) vers l'ouverture de sortie (16), et/ou
d. le distributeur (10) est conçu sous la forme d'un pulvérisateur, et/ou
e. le distributeur est conçu sous la forme d'un inhalateur, en particulier sous la forme d'un distributeur comprenant un récipient qui peut être comprimé pour distribuer une dose du liquide,
f. le distributeur est conçu sous la forme d'un distributeur de gouttes ayant en particulier une surface de formation de gouttes entourant l'ouverture d'évacuation, et/ou
g. le distributeur est conçu sous la forme d'un distributeur nasal, comprenant en particulier un applicateur nasal allongé, à l'extrémité distale duquel est prévue l'ouverture d'évacuation.
